# EUROPÄISCHE PATENTSCHRIFT

(11) **EP 3 106 542 B1**
(45) Veröffentlichungstag und Bekanntmachung des Hinweises auf die Patenterteilung: **15.08.2018**
(21) Anmeldenummer: 16172505.6
(22) Anmeldetag: 01.06.2016
(51) Int. Cl.: C25B 1/00, C25B 1/04, C25B 3/04, C25B 9/18, C25B 15/08, C10G 2/00, C07C 29/151

(54) **VORRICHTUNG ZUR SYNTHESE VON WASSERSTOFFHALTIGEN VERBINDUNGEN**
DEVICE FOR THE SYNTHESIS OF HYDROGENATED COMPOUNDS
DISPOSITIF DE SYNTHÈSE DE LIAISONS CONTENANT DE L'HYDROGÈNE

(30) Priorität: 19.06.2015 DE 102015211391
(43) Veröffentlichungstag der Anmeldung: 21.12.2016
(73) Patentinhaber: Technische Universität Dresden, 01069 Dresden (DE)
(72) Erfinder: LUEDDECKENS, Stefan, 01069 Dresden (DE); LIPPMANN, Wolfgang, 01309 Dresden (DE); UNGER, Sebastian, 01217 Dresden (DE); HURTADO, Antonio, 12347 Berlin (DE)
(74) Vertreter: Rauschenbach, Marion

(56) Entgegenhaltungen:
- EP-A2- 0 497 226
- WO-A2-03/093536
- CN-B- 103 103 556
- US-A- 4 427 504
- US-A1- 2010 086 824

## Beschreibung

Die Erfindung bezieht sich auf die Gebiete des Maschinenbaus und der Chemie und betrifft eine Vorrichtung zur Synthese von wasserstoffhaltigen Verbindungen, wie sie beispielsweise für die Synthese von Methan, Methanol Alkanen, Alkenen oder anderen Kohlenwasserstoffen eingesetzt werden kann.

Die Umwandlungsverfahren unter Nutzung elektrischer Energie zum Antrieb chemischer Reaktionen werden auch als Power-To-Gas (PTG)- oder Power-To-Liquid (PTL)-Verfahren bezeichnet. Das für die verschiedenen Synthesearten benötigte Synthesegas, bestehend aus Wasserstoff und Kohlenstoffmonoxid und/oder Kohlenstoffdioxid, wird auf elektrochemischem Wege durch eine Elektrolyse bereitgestellt (DE 10 2013 017 914 A1). Kohlenstoffmonoxid kann außer durch Elektrolyse von Kohlenstoffdioxid auch aus der Shift-Reaktion, eine Reaktion von Wasserstoff und Kohlenstoffdioxid zu Wasser und Kohlenstoffmonoxid, gewonnen werden (US 8540898 A1). Die Synthesereaktionen sind auch mit Kohlenstoffdioxid durchführbar, wobei die Umsätze deutlich geringer als mit Kohlenstoffmonoxid sind. Dadurch ist eine aufwändigere Trennung von Produkt und nicht umgesetzten Edukten erforderlich. Die Entscheidung für eine Reaktion mit Kohlenstoffdioxid oder Kohlenstoffmonoxid richtet sich einerseits nach den einzelnen Anforderungen der Kohlenwasserstoffsynthesen und andererseits nach der Gewichtung eines möglichst einfachen Aufbaus bei geringen Prozesstemperaturen oder eines möglichst hohen Wirkungsgrads.

Durch diese Verfahren wird elektrische Energie in chemische Energie in Form von Kohlenwasserstoffen umgewandelt. Dazu werden Wasserstoff und Kohlenstoff in ihrem maximalen Oxidationszustand als Wasser und Kohlenstoffdioxid elektrochemisch reduziert und anschließend in einer Synthesereaktion zu Kohlenwasserstoffen umgewandelt.

Derartige Power-to-Gas/Liquid-Verfahren werden derzeit in Pilotanlagen getestet. Wichtigen Hersteller derartiger Anlagen sind die Fa. etogas GmbH (Methansynthese) Stuttgart, die Fa. sunfire GmbH (Dieselherstellung) Dresden oder die Fa. Carbon Recycling International Inc. (Methanolsynthese) Reykjavik.
Allen diesen Verfahren ist gemeinsam, dass sie auf großtechnische chemische Anlagen ausgerichtet sind und möglichst viele, kommerziell verfügbare Anlagenteile aus der chemischen Industrie verwenden. Wasserstoff wird durch alkalische Elektrolyseure oder durch Hochtemperaturelektrolyseure hergestellt, wobei die bei den Syntheseprozessen freiwerdende Abwärme in einigen Fällen rekuperiert wird. Die Rekuperation ist sehr naheliegend, da die Synthesereaktionen exotherme Prozesse sind, während die für die Elektrolyse notwendige Energie auch teilweise thermisch zugeführt werden kann. Derzeit werden Synthesereaktionen ausschließlich mit Kohlenstoffmonoxid umgesetzt, welches ausschließlich über die Shift-Reaktion erzeugt wird.

Als Elektrolyseur wird eine Vorrichtung bezeichnet, in der mit Hilfe elektrischen Stromes eine chemische Reaktion, also eine Stoffumwandlung, herbeigeführt wird: Es findet eine Elektrolyse statt. Entsprechend der Vielfalt an unterschiedlichen Elektrolyten gibt es auch eine Vielzahl von Elektrolyseuren.
Alkalische Elektrolyseure werden als Einzige großindustriell genutzt, sind jedoch aufgrund vergleichbar geringer Wirkungsgrade und der fehlenden Möglichkeit der Rekuperation durch die Abwärmeentstehung auf niedrigem Temperaturniveau für Power-to-Gas/Liquid-Technologien grundsätzlich weniger geeignet als Hochtemperaturelektrolyseure. Für Letztere kann jedoch die Langzeittauglichkeit in kommerziellen Anlagen noch nicht nachgewiesen werden.

Eine Brennstoffzelle ist eine galvanische Zelle, die die chemische Reaktionsenergie eines kontinuierlich zugeführten Brennstoffes und eines Oxidationsmittels in elektrische Energie wandelt.

Eine elektrochemische Zelle kann sowohl als Brennstoffzelle, als auch als Elektrolyseur betrieben werden, indem eine Spannung entweder angelegt wird oder aufgrund eines elektrochemischen Potenzials entsteht. Eine Zelle, die in beide Richtungen funktioniert, wird auch als reversible Brennstoffzelle bezeichnet (Reversible Fuel Cell, RFC). Diese Betriebsweise ist insbesondere möglich, wenn die Katalysatoren im Brennstoffzellen- und Elektrolysebetrieb äquivalent sind, beispielsweise bei Verwendung von Nickel/Ruthenium bei der Herstellung synthetischen Methans. Der Vorteil einer RFC besteht vor allem in der Steigerung der Volllaststunden und damit der wirtschaftlichen Auslastung der Zelle, indem bei günstigen Strompreisen Elektrolyse betrieben wird und bei hohen Strompreisen Strom, beispielsweise aus Erdgas oder synthetischem Methan, erzeugt wird. Mit einer RFC ist also der Betrieb eines Energiespeichers möglich (US 8748052 A1).

Brennstoffzellen und Elektrolyseure können auch durch ihre Arbeitstemperaturen und/oder die verwendeten Elektrolyten unterschieden werden. Als Niedertemperaturzellen werden Zellen mit einer Arbeitstemperatur unterhalb von 200 °C, eher unterhalb von 100 °C, bezeichnet, wie sie bei der Verwendung von Alkalilaugen- oder Polymerelektrolyten üblich sind. Oberhalb dieser Temperaturen sind derzeit nur Schmelzkarbonatzellen als Brennstoffzelle im Einsatz, die bei 500 °C flüssige Karbonate als Elektrolyt verwenden.
Ein bekannter keramischer Elektrolyt ist yttriumstabilisiertes Zirkoniumoxid (YSZ), das ab 700 °C Sauerstoffionen leitet. Durch spezielle Dotierungen, aber auch durch Verwendung anderer Keramiken sind Arbeitstemperaturen zwischen 400 und 700 °C möglich, bei denen diese Keramiken Protonen leiten. Die Nutzung keramischer Zellen mit Arbeitstemperaturen unterhalb von 700 °C mindert das Rekuperationspotenzial nicht, da die Temperaturen der Synthesereaktionen zwischen 170 und maximal 400 °C variieren. Es entsteht jedoch der Vorteil, dass die Prozesse mit wesentlich materialschonenderen Temperaturen, statt der bei YSZ üblichen 900 bis 1000 °C, durchgeführt werden können.

Ein weiterer Vorteil der Nutzung protonenleitender Zellen ist das Vorliegen des gewünschten Produkts, des Wasserstoffs, in sehr hoher Reinheit. Bei oxidleitenden Zellen ist dieser stets mit einem Rest der Edukte, in der Regel Wasser, verunreinigt. Wasser hemmt jedoch die nachfolgenden Synthesereaktionen und muss entfernt werden. Wenn dies durch Auskondensieren geschieht, geht auch die Wärme des Wasserstoffs verloren, welche in einer nachfolgenden Shift-Reaktion als endotherme Reaktion benötigt werden würde. Die Nutzung protonenleitender Elektrolyte in der Elektrolyse bewirkt außerdem das Vorliegen des Wasserstoffs an der Elektrolytgrenzfläche in zunächst atomarer, naszierender Form, wodurch dieser besonders reaktionsfreudig ist.
Aktuelle keramische Hochtemperaturzellen werden in planarer Form zu Stacks zusammengesetzt, welche eine geringe mechanische Stabilität gegenüber thermischen Spannungen aufweisen und leicht zu Bruch gehen, wodurch die Zelle durch den darauf folgenden, möglichen Kurzschluss unbrauchbar wird (US 20140186739 A1). Die Reparatur ist zeitaufwändig und wird oft in Handarbeit durchgeführt Um die thermischen Spannungen gering zu halten, können planare Zellen keine schnellen Lastwechsel durchführen und müssen auch in Stillstandsphasen warm gehalten werden. Wasserstoff- und Sauerstoff-leitende rohrförmige elektrochemische Zellen (rohrförmige Festelektrolyten mit katalysierten Anoden- und Kathodenbeschichtungen auf Innen- bzw. Außenwand), die in unter Druck stehenden, beheizten Hüllrohren untergebracht sind, sind aus dem Stand der Technik bekannt, siehe beispielsweise EP 0 497 226 A2, US 4 427 504 A, CN 103 103 556 B, WO 03/093536 A2, US 2010/086824 A1. Die Aufgabe der vorliegenden Erfindung besteht in der Angabe von Vorrichtung zur Synthese von wasserstoffhaltigen Verbindungen, die einen modularen Aufbau aufweist und in kleinen, austauschbaren Baugruppen einsetzbar ist.

Die Aufgabe wird durch die in den Ansprüchen angegebene Erfindung gelöst. Vorteilhafte Ausgestaltungen sind Gegenstand der Unteransprüche.

Mit der erfindungsgemäßen Lösung wird erstmals eine Vorrichtung zur Synthese von Kohlenwasserstoffen, angegeben, die einen modularen Aufbau aufweist und in kleinen, austauschbaren Baugruppen einsetzbar ist.

Erreicht wird dies durch die erfindungsgemäße Vorrichtung zur Synthese von wasserstoffhaltigen Verbindungen, bestehend aus mindestens einem Hüllrohr. Dieses Hüllrohr besteht vorteilhafterweise aus einem metallischen Werkstoff, wie beispielsweise aus Edelstahl.
Erfindungsgemäß liegt im Hüllrohrinnenraum zur Realisierung der Synthesebedingungen mindestens im Arbeitsstadium ein größerer/höherer Druck als der Umgebungsdruck vor, was durch die konstruktiven Ausführungen der funktionalen Komponenten der Vorrichtung in Form von Rohren vereinfacht ausgeführt werden kann.
Alle zu- oder abgeführten Medien in den Hüllrohrinnenraum müssen damit selbstverständlich unter dem Druck, der im Hüllrohrinnenraum herrscht zu- und abgeführt werden.
Das Hüllrohr kann insbesondere Drücke im Innenraum von 0,5 bis 40 MPa aufnehmen.

Im einem Hüllrohrinnenraum befinden sich mindestens eine Wasserstoffionenleitende rohrförmige elektrochemische Zelle und mindestens eine nicht-Wasserstoffionen-leitende rohrförmige elektrochemische Zelle, oder es liegen mindestens zwei Hüllrohre vor in denen in einem Hüllrohrinnenraum mindestens eine Wasserstoffionen-leitende rohrförmige elektrochemische Zelle und in dem anderen Hüllrohrinnenraum mindestens eine nicht-Wasserstoffionen-leitende rohrförmige elektrochemische Zelle vorhanden ist, wobei alle Zellen von der Innenwandung des Hüllrohres beabstandet angeordnet sind,

Die Wasserstoffionen-leitende rohrförmige elektrochemische Zelle besteht dabei aus einem rohrförmigen Festelektrolyten, bei dem auf mindestens dessen Innen- und Außenwandung eine Beschichtung angeordnet ist, die aus Kathoden- oder Anodenmaterial besteht.

Die Wasserstoffionen-leitende rohrförmige elektrochemische Zelle weist vorteilhafterweise Abmessungen von 1 mm bis 1000 mm Innendurchmesser und 20 mm bis 2000 mm Länge auf.

Als Materialien für den Festelektrolyt der Wasserstoffionen-leitenden rohrförmigen elektrochemischen Zelle können Ceroxid, beta-Aluminat, Calciumzirkonat, Natriumtitanat oder anderen Titanate oder Zirkoniumoxid mit Zusätzen, wie beispielsweise Yttrium und Barium, vorhanden sein.
Im Falle einer Sauerstoffionen-leitenden rohrförmigen elektrochemischen Zelle kann als Material für den Festelektrolyt Ceroxid, yttriumstabilisiertes Zirkoniumoxid oder scandiumstabilisiertes Zirkoniumdioxid vorhanden sein.

Als Anodenmaterial für die elektrochemischen Zellen können Keramik-Metall-Verbunde (Cermet) aus Nickel-yttriumstabilisiertem Zirkonoxid, Nickellegierungen, Platinmetalle, oder Verbunde aus Oxidkeramiken und diesen Metallen und als Kathodenmaterial Lanthanstrontium-manganit, Lanthanstrontium-cobaltitferrit, Nickellegierungen oder Platinmetalle vorhanden sein, wobei mindestens die Innen- und Außenseiten des rohrförmigen Festelektrolyten mindestens teilweise, vorteilhafterweise vollständig, mit dem Anoden- oder Kathodenmaterial beschichtet sind.

Weiter bestehen mindestens bei den Wasserstoffionenleitenden rohrförmigen elektrochemischen Zellen die Oberflächen des rohrförmigen Festelektrolyts mindestens auf der Kathodenseite aus einem Katalysatormaterial und/oder sind ganz oder teilweise damit beschichtet, und/oder ist mindestens teilweise das Anoden- und/oder Kathodenmaterial ein Katalysatormaterial, und/oder ist das Katalysatormaterial auf dem Kathoden- und/oder Anodenmaterial positioniert.

Besonders vorteilhaft ist es, wenn bei den elektrochemischen Zellen die Oberflächen des rohrförmigen Festelektrolyten mit einem Katalysatormaterial mindestens teilweise beschichtet sind oder die Anoden- und/oder Kathodenmaterialien gleichzeitig Katalysatormaterialien sind. Als Katalysatormaterialien können dabei jeweils vorteilhafterweise Platinmetalle, Nickel- oder Eisenverbindungen für die Wasserstoffsynthese, und/oder für die Sauerstoffsynthese, und/oder nickel- oder rutheniumhaltige Materialien für die Methansynthese oder Kupfer-Zinkoxid-Aluminiumoxid-Materialien für die Methanolsynthese, oder kobalthaltige Katalysatoren generell für die Kohlenwasserstoffsynthese vorhanden sein. Vorteilhafterweise sind im Falle der Wasserstoffelektrolyse (ohne Synthese von Kohlenwasserstoffen oder Ammoniak) in der Wasserstoffionen-leitenden rohrförmigen elektrochemischen Zelle die Katalysatormaterialien nur auf der Oberfläche des Festelektrolyten und/oder als Anoden- und/oder Kathodenmaterial und/oder auf der Oberfläche der Anode und/oder Kathode positioniert.
Im Falle einer Synthese von wasserstoffhaltigen Verbindungen in der Wasserstoffionen-leitenden rohrförmigen elektrochemischen Zelle kann das Katalysatormaterial ebenfalls auf der Oberfläche des Festelektrolyten und/oder als Anoden- und/oder Kathodenmaterial und/oder auf der Oberfläche der Anode und/oder Kathode positioniert sein und/oder auch im Raum um die Kathode. Erfindungsgemäß weist der Hüllrohrinnenraum noch mindestens ein Heizelement auf, was beispielsweise ein elektrisches Widerstandsheizelement sein kann.
Es können vorteilhafterweise 2 bis 1000 einzelne Wasserstoffionen-leitende rohrförmige elektrochemische Zellen oder als Bündel solcher Zellen in einem Hüllrohr oder innerhalb mehrerer Hüllrohre zusammengefasst sein. Im Falle von mehreren Hüllrohren können dann auch deutlich mehr als 1000 einzelne Wasserstoffionenleitende rohrförmige elektrochemische Zellen in den Hüllrohren vorhanden und/oder auch als Bündel zusammengefasst sein. Auch mehrere Hüllrohre können zu Modulen zusammengefasst sein und hinsichtlich ihrer Funktionsweise zusammengesetzt werden.
Diese einzelnen oder gebündelten Zellen und/oder Hüllrohre können mindestens teilweise oder vollständig gemeinsame Zuführungen und mindestens teilweise oder vollständig gemeinsame Abführungen aufweisen und ebenso mit einer gemeinsamen Stromversorgung und Steuer- und Regelmechanismen verbunden sein, und bei mehreren Wasserstoffionen-leitenden rohrförmigen elektrochemischen Zellen können die Rohre oder Bündel über- und/oder hintereinander im Hüllrohrinnenraum angeordnet sein.

Erfindungsgemäß sind weiter mindestens bei den Wasserstoffionen-leitenden rohrförmigen elektrochemischen Zellen auf der Anodenseite der Zelle eine Zuführung für Wasser oder Wasserdampf vorhanden und auf der Kathodenseite der Zelle eine Zuführung für Kohlenstoffdioxid oder Kohlenstoffmonoxid oder Stickstoff. Weiter sind erfindungsgemäß mindesten bei den Wasserstoffionen-leitenden rohrförmigen elektrochemischen Zellen auf der Anodenseite eine Abführung für Sauerstoff und auf der Kathodenseite eine Abführung für Kohlenwasserstoffe oder Ammoniak vorhanden, wobei ebenfalls eine Abführung von nicht umgesetzten Edukten und/oder Reaktionsprodukten vorhanden ist,
Ebenso ist es erfindungsgemäß von besonderer Bedeutung, dass die mindestens eine Wasserstoffionen-leitende rohrförmige elektrochemische Zelle und die mindestens eine nicht-Wasserstoffionenleitende rohrförmige elektrochemische Zelle innerhalb eines Hüllrohres oder innerhalb unterschiedlicher Hüllrohre funktionell in Reihe und/oder parallel geschaltet sind.
Wenn einige oder alle Wasserstoffionen-leitenden rohrförmigen elektrochemischen Zellen und einige oder alle nicht-Wasserstoffionen-leitenden rohrförmigen elektrochemischen Zellen funktionell parallel geschaltet sind, so liegen getrennte Zuführungen und Abführungen für die Ausgangs- und Endprodukte jeder Zelle vor. Sind einige oder alle Wasserstoffionen-leitenden rohrförmigen elektrochemischen Zellen und einige oder alle nicht-Wasserstoffionen-leitenden rohrförmigen elektrochemischen Zellen funktionell in Reihe geschaltet, so sind die Zuführungen gleichzeitig die Abführungen oder die Abführungen gleichzeitig die Zuführungen von anderen Zellen. Zusätzlich können vorhanden sein Wärmeübertrager oder Wärmetauscher zur Rekuperation von Abwärme exothermer Prozesse, die in den Hüllrohren ablaufen, die Abwärme aus Synthesereaktion, Abwärme des Produktstromes, Abwärme der Zwischenprodukte aus der Elektrolyse und/oder der Shift-Reaktion, und/oder Verdichterabwärme nutzen können und/oder es können Trennvorrichtungen sein, wie Vorrichtungen zur Trennung von Haupt- und Nebenprodukten, wie Wasser und nicht umgesetzte Edukte, beispielsweise durch Destillation, Kondensation oder Membranverfahren, oder zur Abtrennung von Wasserdampf und/oder es können Vorrichtungen zur Synthese und/oder zur Verarbeitung von Wasser, Wasserstoff, Kohlenstoffmonoxid und/oder Kohlenstoffdioxid sein. Besonders vorteilhafterweise sind die Zuführungen für Wasser oder Wasserdampf auf der Anodenseite einer Wasserstoffionen-leitenden rohrförmigen elektrochemischen Zelle mit den Abführungen von Wärmeübertragern oder Wärmetauschern zur Rekuperation von Abwärme exothermer Prozesse funktionell verbunden, und die Zuführungen für Kohlenstoffmonoxid oder auf der Kathodenseite der Wasserstoffionen-leitenden rohrförmigen elektrochemischen Zelle sind mit den Abführungen einer Sauerstoffionen-leitenden rohrförmigen elektrochemischen Zelle funktionell verbunden, und die Abführungen für Sauerstoff auf der Anodenseite einer Wasserstoffionen-leitenden rohrförmigen elektrochemischen Zelle sind mit der Zuführung einer Sauerstoffionen-leitenden rohrförmige elektrochemische Zelle funktionell verbunden.
Die erfindungsgemäße modulare Bauweise ergibt sich insbesondere dadurch, dass ein oder mehrere Wasserstoffionen-leitende rohrförmige elektrochemische Zellen als Bündel in einem Hüllrohr angeordnet sind und dass diese als Module zu Anlagen in beliebiger Größe zusammen angeordnet und betrieben werden können.
Ebenfalls können einzelne Wasserstoffionen-leitende rohrförmige elektrochemische Zellen und einzelne nicht-Wasserstoffionen-leitende rohrförmige elektrochemische Zellen in unterschiedlichen Hüllrohren vorhanden sein und dann in paralleler Funktionsweise zu einem Bündel zusammengefasst, oder hinsichtlich ihrer Funktionsweise als Module in Reihe geschalten sein.
Damit ist eine flexible Anordnung der Vorrichtung und eine flexible Verfahrensdurchfühung möglich, die auch zu einer hohen Ausfallsicherheit und Wartungsfreundlichkeit der Gesamtanlage führt, da insbesondere auch die Möglichkeit der einzelnen Ansteuerbarkeit der Module besteht.

Es können auch vorteilhafterweise mehrere rohrförmige Zellen oder Bündel von rohrförmigen Zellen in einem Hüllrohr angeordnet sein, wobei die einzelnen Zellen aus verschiedenen Festelektrolyt-, Anoden- und/oder Kathodenmaterialien bestehen können und somit verschiedene Stoffe, wie Wasserdampf oder Kohlenstoffdioxid oder Stickstoff, verarbeiten und gewünschte wasserstoffhaltige Verbindungen optimaler Zusammensetzung erzeugen können.

Weiterhin können vor und/oder nach und/oder zwischen der einen oder mehreren Wasserstoffionen-leitenden rohrförmigen elektrochemischen Zellen oder Bündeln weitere Vorrichtungen im Hüllrohrinneren angeordnet sein, wie Vorrichtungen zur Synthese und/oder zur Verarbeitung von Wasser, Wasserstoff, Kohlenstoffmonoxid und/oder Kohlenstoffdioxid oder Stickstoff.
Ebenso können auch außerhalb des Hüllrohres ein oder mehrere weitere Vorrichtungen angeordnet sein, die mit Vorrichtungen im Innenraum des Hüllrohres direkt verbunden sind, wie vorteilhafterweise Wärmeübertragungsvorrichtungen oder Wärmetauscher zur Rekuperation von Abwärme exothermer Prozesse im Hüllrohr, wie der Abwärme der Synthesereaktion, der Abwärme des Produktstromes, der Abwärme der Zwischenprodukte aus der Elektrolyse und/oder der Verdichterabwärme, und/oder Trennvorrichtungen, wie Vorrichtungen zur Abtrennung von Wasserdampf oder Kondensation von Ammoniak.

Bei der erfindungsgemäßen Wasserstoffionen-leitenden rohrförmigen elektrochemischen Zelle im Hüllrohrinnenraum wird, je nach Vorhandensein des Anoden- und Kathodenmaterials, auf der Innen- und Außenseite des Festelektrolyten, die Leitung der Wasserstoffionen entweder in die Rohrinnenseite oder auf die Rohraußenseite der Wasserstoffionen-leitenden rohrförmigen elektrochemischen Zelle realisiert.
Vorteilhafterweise ist jeweils oder auch nur auf einer Seite, also auf Seiten der Kathode oder der Anode, ein Katalysatormaterial vorhanden. Das Katalysatormaterial entspricht üblichen Katalysatoren für die verschiedenen Kohlenwasserstoffsynthesen, wie beispielsweise nickel- oder rutheniumhaltige Katalysatoren für die Methansynthese oder Kupfer-Zinkoxid-Aluminiumoxid-Katalysatoren für die Methanolsynthese.

Von besonderer Bedeutung ist dabei die Anordnung der Katalysatoren, die erfindungsgemäß erstmals in direkter räumlicher Nähe der elektrochemischen Zelle, entweder direkt an der Außen- und/oder Innenseite des rohrförmigen Festelektrolyten (je nach Fließrichtung der Ionen) oder auf anderen Vorrichtungen, wie beispielsweise auf Sauerstoffionen-leitenden rohrförmigen elektrochemischen Zellen, innerhalb des Hüllrohres angeordnet sind.
Die Funktionsweise der erfindungsgemäßen Vorrichtung ist Folgende.
Die in dem Hüllrohrinnenraum positionierte Wasserstoffionen-leitenden rohrförmige elektrochemische Zelle weist auf der Anodenseite der Zelle eine Zuführung für Wasserdampf und eine Abführung für Sauerstoff auf. Über den Festelektrolyten erfolgt die Leitung der Wasserstoffionen auf die Kathodenseite der Zelle, auf der dann Wasserstoff vorliegt.
Der besondere Vorteil der vorliegenden Erfindung besteht beim Einsatz von Wasserstoffionen-leitenden elektrochemischen Zellen darin, dass reiner Wasserstoff bereitgestellt wird und kein Reinigungsprozess erforderlich ist.
Auf der Kathodenseite der Wasserstoffionen-leitenden rohrförmigen elektrochemischen Zelle ist eine Zuführung für Kohlenstoffmonoxid und eine Abführung für Kohlenwasserstoffe, und möglicherweise nicht reaktiv umgesetzte Edukte oder andere Reaktionsprodukte. Die Reaktion auf der Kathodenseite der Wasserstoffionen-leitenden rohrförmigen elektrochemischen Zelle erfolgt zwischen dem erzeugten Wasserstoff und dem zugeführten Kohlenstoffmonoxid. Die Reaktion führt zur Synthese von wasserstoffhaltigen Verbindungen, wie einerseits zu Kohlenwasserstoffen, z.B. wie Methan, Methanol, Alkane, Alkene, Otto- oder Dieselkraftstoffe. Dabei kann erfindungsgemäß das Anodenmaterial auf der Innenseite des rohrförmigen Festelektrolyten angeordnet sein. Dann wird vorteilhafterweise in den Rohrinnenraum der Zelle Wasserdampf auf einer Seite zu- und auf der anderen Seite Sauerstoff abgeführt. Der Wasserstoff entsteht dann auf der Außenseite des rohrförmigen Festelektrolyten. Dann werden in den Raum um die Außenseite des rohrförmigen Festelektrolyten Kohlenstoffmonoxid zu- und die entstehenden wasserstoffhaltigen Verbindungen und nicht reaktiv umgesetzte Edukte oder andere Reaktionsprodukte abgeführt.
Sofern das Kathodenmaterial auf der Innenseite des rohrförmigen Festelektrolyten angeordnet ist, erfolgen die Zu- und Abführungen der genannten Materialien gerade umgekehrt, also Kohlenstoffmonoxid werden in den Rohrinnenraum der Zelle zu- und die entstehenden wasserstoffhaltigen Verbindungen und nicht reaktiv umgesetzte Edukte oder andere Reaktionsprodukte abgeführt, und Wasserdampf wird in den Raum um die Außenseite des rohrförmigen Festelektrolyten auf einer Seite zu- und auf der anderen Seite Sauerstoff abgeführt.

Von besonderem Vorteil für die erfindungsgemäße Lösung ist, dass neben einer oder mehreren Wasserstoffionen-leitenden rohrförmigen elektrochemischen Zellen mindestens eine oder mehrere Sauerstoffionen-leitende rohrförmige elektrochemische Zellen in dem Hüllrohr vorhanden sind. In diesem Fall kann der bei der oder den Wasserstoffionen-leitenden rohrförmigen elektrochemischen Zellen erzeugte Sauerstoff auf die Anodenseite der Sauerstoffionen-leitenden rohrförmigen elektrochemischen Zellen geführt werden. Auf der Kathodenseite der Sauerstoffionen-leitenden rohrförmigen elektrochemischen Zellen kann Kohlenstoffdioxid zugeführt werden, welches zu Kohlenstoffmonoxid reagiert, das wiederum in den Reaktionsraum an der oder den Wasserstoffionen-leitenden rohrförmigen elektrochemischen Zellen über die Zuführung eingebracht werden kann. Wie bereits bei den Wasserstoffionen-leitenden rohrförmigen elektrochemischen Zellen beschrieben, kann die Leitung der Sauerstoffionen erfindungsgemäß auch hier entweder von Innen nach Außen des rohrförmigen Festelektrolyten der Zelle oder von außen nach innen erfolgen.
Eine Verdichtung des zugeführten Wasserdampfs auf den im Hüllrohr vorhandenen Druck kann bereits, zumindest teilweise, durch die Verdampfung des dazu notwendigen Speisewassers erreicht werden, bei welcher oben beschriebene Abwärmeströme rekuperiert werden können. Eine weitere, beispielsweise mechanische Verdichtung ist vom gewünschten Reaktionsprodukt und den damit verbundenen Reaktionsbedingungen abhängig.
Eine Verdampfung und Verdichtung vorteilhafterweise außerhalb des Hüllrohrs wird durch ein Wärmeübertragermedium realisiert, mit welchem der Wasserdampf oder das Wasser abgeschieden wird und die dabei gewonnene Energie auf die Ausgangsstoffe übertragen werden kann. Alternativ entspricht das Wärmeübertragungsmedium dem Speisewasser, welches an den verschiedenen Stationen der Wärmeübertragung erhitzt und verdampft wird. Die Nutzung eines separaten Wärmeübertragungsmediums ist vom Druck im Hüllrohr abhängig. Bei hohen Drücken, wie bei der Methanolsynthese (ca. 5 MPa), ist eine vollständige Verdampfung des Wassers auf diesem Druckniveau nicht möglich, sodass die Wärme über ein Medium zum Speisewasser übertragen wird, welches dann zunächst bei geringerem Druck vorliegen kann.
Auch bei den Sauerstoffionen-leitenden rohrförmigen elektrochemischen Zellen kann vorteilhafterweise Katalysatormaterial vorhanden sein, dass mindestens den Elektrolyseprozess unterstützt, wie beispielsweise Nickel oder Platinmetalle.
Die Katalysatormaterialien der Syntheseprozesse können bei den erfindungsgemäß eingesetzten Zellen sowohl direkt auf die Elektroden aufgebracht sein, um Reaktionen direkt an den Grenzflächen zu ermöglichen, als auch im Hohlraum in verschiedenartigen Varianten, beispielsweise als Pellets, Schaum oder Wolle, angebracht sein.
Zur Verbesserung aller Reaktionen, sowohl der Synthese von Wasserstoff oder Sauerstoff als auch von wasserstoffhaltigen Verbindungen oder Kohlenstoffmonoxid können die jeweiligen funktionalen Oberflächen des Festelektrolyten strukturiert sein, was beispielsweise mit einer Laserbehandlung, einer Ionenbestrahlung oder anderen Verfahren realisiert werden kann.
Als Edukte sollen im Rahmen dieser Erfindung sowohl der durch die Zellen synthetisierte Wasserstoff verstanden werden, aber auch zugeführtes Kohlenstoffdioxid und/oder Kohlenstoffmonoxid. Als weitere Reaktionsprodukte sollen im Rahmen dieser Erfindung beispielsweise Wasser verstanden werden, wobei bei den ablaufenden Reaktionen üblicherweise auch kleine Mengen andere, als die gewünschten Stoffe entstehen können, welche überwiegend selbst Kohlenwasserstoffe sind.

Die indirekte Beheizung des Hüllrohrs ist für die Wärmerückgewinnung und Heizung der gesamten Vorrichtung von außen, sowie der Kühlung der Synthesereaktion besonders vorteilhaft. Das Wärmeübertragungsmedium kann gleichzeitig das Speisewasser für die Elektrolyse oder auch ein eigener Wärmeübertragungskreislauf sein. Dies ist von der Herstellung des gewünschten Endproduktes und insbesondere von der dafür notwendigen Temperatur abhängig, da es vorteilhaft ist, das Speisewasser durch die Abwärme zu verdampfen.
Eine Wärmerückgewinnung wird sinnvollerweise erfindungsgemäß stets angestrebt. Die Nutzung der Abwärme für die Vorwärmung und, wenn bei der einzelnen Verfahrensführungen möglich, der Verdampfung des Speisewassers ist die wesentliche Rückgewinnungsstufe. Auch die Vorwärmung des Kohlenstoffdioxids, soweit es durch Verdichtung nicht bereits Betriebstemperatur aufweist, kann erfindungsgemäß durchgeführt werden. Bei Verfahrensführungen mit hohen Prozessdrücken kann Kohlenstoffdioxid durch Verdichtung eine Temperatur oberhalb der optimalen Reaktionstemperatur aufweisen. In diesem Fall kann das Kohlenstoffdioxid in Verbindung mit einer Wärmerückgewinnung abgekühlt werden. Die im Produktstrom enthaltenen, nicht umgesetzten Edukte der Reaktionen werden entwässert und zurückgeführt, wobei ein kleiner Anteil dieses Rezyklats entsorgt werden kann, um die Anreicherung von Störstoffen zu verhindern. Die zurückgeführten Edukte werden durch Wärmerückgewinnung ebenfalls vorgewärmt. Eine weitere Wärmequelle stellt der noch warme Produktstrom dar. Dessen Wärme wird ebenfalls zur Vorwärmung der Ausgangsstoffe und/oder Edukte genutzt. Zudem können die Zwischenprodukte aus Elektrolyse und/oder Shift-Reaktion eine Temperatur oberhalb der für die nachfolgende Reaktion notwendige Temperatur aufweisen. Durch Abkühlung dieser Zwischenprodukte kann weitere Wärme rekuperiert werden.

Verdichter können für die Druckerhöhung der Ausgangsstoffe und/oder Reaktionsprodukte und/oder Edukte, beispielsweise wie Wasser und Kohlenstoffdioxid, verwendet werden. Vorteilhafterweise wird Wasser durch Rekuperation bereits bei Prozessdruck verdampft. Dies ist insbesondere bei Verfahren, die einen geringen Prozessdruck erfordern, wie bei der Methansynthese, möglich. Bei Verfahren, bei denen ein hoher Prozessdruck erforderlich ist, wie bei der Methanolsynthese, kann eine Nachverdichtung eingesetzt werden.

Ein besonderer Vorteil der erfindungsgemäßen Lösung besteht in seinem modularen Aufbau in kleinen, austauschbaren Baugruppen. So können mehrere einzelne Zellen oder mehrere Zellen als Bündel in einem oder mehreren Hüllrohren modular zusammengeschaltet werden und die Peripheriekomponenten, wie Wasser- und Kohlenstoffdioxidverdichter, Dampfspeicher, Wärmeübertragereinheiten und elektrische Baugruppen gemeinsam genutzt werden. Ebenso können innerhalb des Hüllrohres oder mit unterschiedlichen Hüllrohren dann unterschiedliche Prozessführungen realisiert werden.
Der modulare Aufbau bietet den weiteren Vorteil, dass einzelne Baugruppen bei Beschädigung oder Ausfall ausgetauscht werden und die gesamte Vorrichtung dann weiterarbeiten kann.

Ebenso ist der rohrförmige Aufbau der erfindungsgemäßen Vorrichtung ein Vorteil, da schnelle Lastwechsel möglich sind und die mechanische Festigkeit deutlich höher als bei planaren Vorrichtungen ist.

Durch den Einsatz von rohrförmigen Zellen mit geringen Innendurchmessern kann auch der bisherige Nachteil von rohrförmigen elektrochemischen Zellen in Form des geringen Umsatzes des Stoffstroms im Zentrum von deren Rohren behoben werden.

Grundsätzlich kann auch bei größeren Rohrinnendurchmessern bei den erfindungsgemäßen Zellen der Stoffstrom durch Einbauten oder durch teilweise radiale Einströmung und Verwirbelung des Stoffstroms besser umgesetzt werden. Die Verwirbelung führt dabei auch zu einer reaktiveren Grenzschicht. Unter anderem kann ein zentraler Strom durch Einbau eines zentralen Körpers verhindert werden.

Insgesamt besteht durch die erfindungsgemäße Lösung bei dieser Vorrichtung eine höhere Ausfallsicherheit, eine einfachere Wartung, eine höher Teillastfähigkeit durch Teilabschaltung und geringere Investitionskosten durch kleinere Anlage und die Möglichkeit kleiner, dezentraler Anlagen.

Insbesondere die Kombination von Wasserstoffionen-leitender Wasser-Elektrolyse und Sauerstoffionen-leitender Kohlenstoffdioxid-Elektrolyse oder der direkten Verwendung von Kohlenstoffdioxid zur Synthese von Kohlenwasserstoffen ist mit minimalem Prozessaufwand und bei moderaten Temperaturen durchführbar. Auf diese Art und Weise sind Elektrolyse von mindestens Wasserstoff und auch Sauerstoff und weiterhin die Synthese von kohlenwasserstoffhaltigen Verbindungen erstmals kompakt in einem gemeinsamen Hüllrohr untergebracht.

Nachfolgend wird die Erfindung an mehreren Ausführungsbeispielen näher erläutert.

### Referenzbeispiel 1: Methansynthese mit Wasserstoffionen-leitender Wasserelektrolyse

Ein Hüllrohr aus Edelstahl mit einem Durchmesser von 80 mm beinhaltet ein Bündel aus 53 Wasserstoffionen-leitenden rohrförmigen elektrochemischen Zellen. Die 53 Wasserstoffionen-leitenden rohrförmigen elektrochemischen Zellen weisen einen rohrförmigen Festelektrolyten aus bariumdotiertem yttriumstabilisiertem Zirkoniumoxid mit jeweils einer Länge von 130 mm und einen Innendurchmesser von 1 mm auf. Auf der Innenseite des rohrförmigen Festelektrolyten ist eine 1 mm dicke Schicht aus Nickelschaum als Anodenmaterial mit einer 2 mg/cm² starken Schicht aus Platin als Katalysatormaterial aufgebracht. Auf der Außenseite des Festelektrolyten ist eine 1 mm dicke Schicht aus Nickelschaum als Kathodenmaterial und eine 2 mg/cm² starke Schicht aus Platin als Katalysatormaterial aufgebracht. In das Hüllrohr und in den Innenraum der 53 Wasserstoffionen-leitenden rohrförmigen elektrochemischen Zellen sind Zuführungen für Wasserdampf und aus dem Innenraum heraus Abführungen für Sauerstoff vorhanden. Auf der Außenseite der 53 Wasserstoffionen-leitenden rohrförmigen elektrochemischen Zellen wird Kohlenstoffdioxid zugeführt. Wasserstoff, Wasserdampf, Methan und nicht umgesetzte Edukte, wie Wasser und Kohlenstoffdioxid, sowie entstandenes Wasser und Reststoffe, wie Ethan, Ethen und Spuren anderer Kohlenwasserstoffe und entstandener Sauerstoff werden über Abführungen auf der Außenseite der Zellen aus dem Hüllrohr herausgeführt.

Das Hüllrohr weist im Inneren einen Druck von 1 MPa auf. Alle zu- und abgeführten Medien werden außerhalb des Hüllrohrs auf diesen Druck verdichtet und dann erst in das Hüllrohr zugeführt.

Das Hüllrohr besitzt Durchführungen von elektrischen Anschlüssen für die Wasserstoffionen-leitenden elektrochemischen Zellen, sowie elektrische Heizelemente vor diesen Zellen. Aus einem Dampfspeicher wird Wasserdampf über das Hüllrohr in einen Verteiler und damit in die Wasserstoffionen-leitenden rohrförmigen elektrochemischen Zellen geleitet und zu Wasserstoff und Sauerstoff umgesetzt. Der Sauerstoff wird aus der Vorrichtung abgeführt und nimmt an keinen weiteren Prozessschritten teil. Der Wasserstoff strömt auf die Außenseite der Zellen in den Raum zwischen Hüllrohrinnenwandung und Außenseite der Wasserstoffionenleitenden elektrochemischen Zellen. In diesen Raum wird ebenfalls das zugeführte Kohlenstoffdioxid geleitet. Das Wasserstoff-Kohlenstoffdioxid-Gemisch wird dann hier zu Methan und Wasser umgesetzt, wobei der Synthesebereich mit einer Schüttung aus Nickel-Aluminiumoxid-Katalysatorpellets gefüllt ist.

Die Reaktion wird durch die an der Außenseite der elektrochemischen Zellen, sowie durch das im Raum zwischen den 53 Wasserstoffionen-leitenden rohrförmigen elektrochemischen Zellen vorhandene Katalysatormaterial katalysiert. Der aus Methan und Wasser bestehende Produktstrom, sowie nicht umgesetzter Wasserstoff und Kohlenstoffdioxid werden aus dem Hüllrohr abgeführt und durch Membranverfahren getrennt. Nicht umgesetzter Wasserstoff und Kohlenstoffdioxid werden in das Hüllrohr auf die Außenseiten der Zellen zurückgeführt.

Speisewasser wird durch eine Pumpe auf den im Inneren des Hüllrohres herrschenden Druck verdichtet und durch entstehende Produktströme, nicht umgesetzte Edukte und ein elektrisches Heizelement außerhalb des Hüllrohres auf Siedetemperatur erwärmt. Dieses auf Siedetemperatur erwärmte Wasser wird in den Bereich um die Zellen geführt, wo es von den dort vorhandenen Medien die exotherme Reaktionsenergie aus den Reaktionen aufnimmt und dadurch das Wasser verdampft wird. Der Wasserdampf wird einem externen Dampfspeicher zugeführt. Dieser ist, ebenso wie die elektrochemischen Zellen durch Widerstandsheizer elektrisch beheizt und erwärmt damit den Wasserdampf auf die für die Wasserelektrolyse erforderliche Temperatur. Dieser Wasserdampf wird dem Hüllrohr und dem Innenraum der 53 Wasserstoffionen-leitenden rohrförmigen elektrochemischen Zellen zugeführt.

Das zugeführte Kohlenstoffdioxid wird durch einen elektrisch betriebenen Verdichter auf den Druck im Hüllrohr verdichtet. Alle Vorrichtungen außerhalb des Hüllrohres sind einmalig vorhanden und können weitere Hüllrohre in einem modularen Aufbau versorgen.
Die Hüllrohre können auch in umgekehrter Reihenfolge als Brennstoffzelle betrieben werden, wobei Methan anstelle von Kohlenstoffdioxid verdichtet wird.

### Beispiel 2: Methanolsynthese mit Wasserstoffionen-leitender Wasserelektrolyse und Sauerstoffionen-leitender Kohlenstoffdioxidelektrolyse

Ein Hüllrohr mit 53 Wasserstoffionen-leitenden rohrförmigen elektrochemischen Zellen, wie in Beispiel 1 angegeben, weist weiterhin eine Bündel aus 48 Sauerstoffionen-leitenden rohrförmigen elektrochemischen Zellen auf.

Die 48 Sauerstoffionen-leitenden rohrförmigen elektrochemischen Zellen weisen einen rohrförmigen Festelektrolyten aus yttriumstabilisiertem Zirkoniumoxid mit jeweils einer Länge von 130 mm und einen Innendurchmesser von 1 mm auf. Auf der Innenseite des rohrförmigen Festelektrolyten ist eine 1 mm dicke Schicht aus Nickelschaum als Anodenmaterial mit einer 2 mg/cm² starken Schicht aus Platin als Katalysatormaterial aufgebracht. Auf der Außenseite des Festelektrolyten ist eine 1 mm dicke Schicht aus Nickelschaum als Kathodenmaterial und eine 2 mg/cm² starke Schicht aus Platin als Katalysatormaterial aufgebracht.
Aus dem Innenraum der 48 Sauerstoffionen-leitenden rohrförmigen elektrochemischen Zellen heraus sind Abführungen für Sauerstoff vorhanden. Auf der Außenseite der 48 Sauerstoffionen-leitenden rohrförmigen elektrochemischen Zellen wird Kohlenstoffdioxid zugeführt. Methanol und nicht umgesetzte Edukte, wie Wasser, Kohlenstoffmonoxid und Kohlenstoffdioxid, sowie Nebenprodukte wie Ethanol oder Formaldehyd werden über eine Abführung auf der Außenseite der Zellen aus dem Hüllrohr herausgeführt. Auch der Sauerstoff wird zusammen mit dem Sauerstoff aus den Wasserstoffionen-leitenden Zellen aus dem Hüllrohr herausgeführt.

Das Hüllrohr besitzt Durchführungen von elektrischen Anschlüssen für die elektrochemischen Zellen, sowie elektrische Heizelemente vor diesen Zellen. Aus einem Dampfspeicher wird Wasserdampf über das Hüllrohr in einen Verteiler und damit in die Wasserstoffionen-leitenden rohrförmigen elektrochemischen Zellen geleitet und zu Wasserstoff und Sauerstoff umgesetzt. Der Sauerstoff wird in die Zuführung in den Innenraum der 48 Sauerstoffionen-leitenden rohrförmigen elektrochemischen Zellen geleitet, um gemeinsam mit dort entstehendem Sauerstoff abgeführt zu werden. Das auf die Außenseite der 48 Sauerstoffionen-leitenden rohrförmigen elektrochemischen Zellen zugeführte Kohlenstoffdioxid wird zu Kohlenstoffmonoxid und Sauerstoff reduziert. Die Umsetzung beträgt 80%, weshalb ein Gemisch mit einen Kohlenstoffmonoxid zu Kohlenstoffdioxid im Verhältnis von 4:1 vorliegt. Der Sauerstoff aus Wasser- und Kohlenstoffdioxidelektrolyse wird aus der Vorrichtung abgeführt und nimmt an keinen weiteren Prozessschritten teil. Der Wasserstoff strömt in einen Raum zwischen Hüllrohrinnenwandung und Außenwandung der Wasserstoffionen-leitenden elektrochemischen Zellen und wird einem Wärmetauscher zugeführt und auf Methanolsynthesetemperatur gekühlt. Das Gemisch aus Kohlenstoffdioxd und Kohlenstoffmonoxid strömt aus dem Innenraum der Sauerstoffionen-leitenden rohrförmigen elektrochemischen Zellen in einen Wärmetauscher und kühlt dort ebenfalls auf Methanolsynthesetemperatur ab.

Der Wasserstoff und der Gemischtstrom aus Kohlenstoffdioxid und Kohlenstoffmonoxid werden in dem der Elektrolyse nachgeordneten Reaktionsbereich zugeführt und zu Methanol und Wasser umgesetzt. Die Reaktion wird durch die eingebrachten Katalysatoren als Schüttung von Kupfer-Zinkoxid-Aluminiumoxid-Pellets katalysiert. Der aus Methanol und Wasser bestehende Produktstrom, sowie nicht umgesetzter Wasserstoff, Kohlenstoffmonoxid und Kohlenstoffdioxid werden aus dem Hüllrohr abgeführt und durch Kondensation getrennt. Nicht umgesetzter Wasserstoff, Kohlenstoffmonoxid und Kohlenstoffdioxid werden zum Reaktionsbereich zurückgeführt. Speisewasser wird durch eine Pumpe auf den im Hüllrohr insgesamt herrschenden Druck von 10 MPa verdichtet und durch entstehende Produktströme und nicht umgesetzte Edukte in der Kondensationskühlung außerhalb des Hüllrohres erwärmt. Das Speisewasser wird weiter dem Hüllrohr zugeführt und durch Umströmung des Bereichs der exothermen Methanolreaktion, sowie durch Kühlung der Elektrolyseprodukte in den Wärmetauschern weiter erwärmt. Das erwärmte Speisewasser wird anschließend einem externen, elektrisch beheizten Dampferzeuger mit Dampfspeicher zugeführt. Dieser ist, ebenso wie die elektrochemischen Zellen durch Widerstandsheizer elektrisch beheizt und erzeugt und überhitzt Wasserdampf auf die für die Wasserelektrolyse erforderliche Temperatur. Dieser Wasserdampf wird dem Hüllrohr und den 53 Wasserstoffionen-leitenden rohrförmigen elektrochemischen Zellen zugeführt. Das zugeführte Kohlenstoffdioxid wird durch einen elektrisch betriebenen Verdichter auf den im Hüllrohr herrschenden Druck verdichtet.
Alle Vorrichtungen außerhalb des Hüllrohres sind einmalig vorhanden und können weitere Hüllrohre in einem modularen Aufbau versorgen.

### Referenzbeispiel 3: Ammoniaksynthese mit Wasserstoffionen-leitender Wasserelektrolyse

Eine Vorrichtung gemäß Beispiel 1 wird zur Ammoniaksynthese eingesetzt. Dabei sind die zugeführten Stoffe Wasser und Stickstoff (statt Kohlenstoffdioxid), die auf einen Druck von 30 MPa verdichtet worden sind, was auch dem Rohrinnendruck im Hüllrohr entspricht. Das Wasser wird durch einen separaten Kühlwasserkreislauf, welcher den Synthesebereich umströmt, nach seiner Verdichtung erwärmt und in einem elektrisch beheizbaren Dampfspeicher verdampft. Wasserstoff und Stickstoff liegen im Synthesebereich in einem Verhältnis von 3:1 vor. Im Synthesebereich ist eine Schüttung aus Katalysatorpellets aus den für diese Synthese üblichen Materialien auf Basis von Magnetit mit Kobalt als Promotor vorhanden. Das entstehende Ammoniak wird einem Kondensator zugeführt, wo es dessen Wärme an das Speisewasser übertragen und anschließend in flüssiger Form gelagert wird.

## Patentansprüche

1. Vorrichtung zur Synthese von wasserstoffhaltigen Verbindungen,
- bestehend aus mindestens einem Hüllrohr, welches im Hüllrohrinnenraum mindestens eine Wasserstoffionen-leitende rohrförmige elektrochemische Zelle und mindestens eine nicht-Wasserstoffionen-leitende rohrförmige elektrochemische Zelle aufweist, die jeweils von der Innenwandung des Hüllrohres beabstandet angeordnet sind, wobei die Wasserstoffionen-leitende rohrförmige elektrochemische Zelle aus einem rohrförmigen Festelektrolyten besteht, und auf mindestens der Innen- und Außenwandung des rohrförmigen Festelektrolyten eine Beschichtung angeordnet ist, die aus Kathoden- oder Anodenmaterial besteht,
- oder bestehend aus mindestens zwei Hüllrohren, von denen in einem Hüllrohrinnenraum mindestens eine Wasserstoffionen-leitende rohrförmige elektrochemische Zelle und in dem anderen Hüllrohrinnenraum mindestens eine nicht-Wasserstoffionenleitende rohrförmige elektrochemische Zelle vorhanden ist, wobei beide Zellen jeweils von der Innenwandung des Hüllrohres beabstandet angeordnet sind, und wobei die Wasserstoffionen-leitende rohrförmige elektrochemische Zelle aus einem rohrförmigen Festelektrolyten besteht, und auf mindestens der Innen- und Außenwandung des rohrförmigen Festelektrolyten eine Beschichtung angeordnet ist, die aus Kathoden- oder Anodenmaterial besteht,
- wobei im Hüllrohrinnenraum jedes Hüllrohres mindestens im Arbeitsstadium ein größerer Druck als Umgebungsdruck vorhanden ist,
- und wobei im Hüllrohrinnenraum mindestens eines Hüllrohres ein Heizelement vorhanden ist,
- und wobei mindestens bei den Wasserstoffionenleitenden rohrförmigen elektrochemischen Zellen die Oberflächen des rohrförmigen Festelektrolyts mindestens auf der Kathodenseite ein Katalysatormaterial aufweisen und ganz oder teilweise damit beschichtet sind, und/oder mindestens teilweise das Anoden- und Kathodenmaterial ein Katalysatormaterial ist, und das Katalysatormaterial auf dem Kathoden- und/oder Anodenmaterial positioniert ist,
- und wobei mindestens bei den Wasserstoffionen-leitenden rohrförmigen elektrochemischen Zellen auf der Anodenseite der Zelle eine Zuführung für Wasser oder Wasserdampf vorhanden ist, und auf der Kathodenseite der Zelle eine Zuführung für Kohlenstoffmonoxid vorhanden ist,
- und wobei mindestens bei den Wasserstoffionen-leitenden rohrförmigen elektrochemischen Zellen auf der Anodenseite eine Abführung für Sauerstoff und auf der Kathodenseite eine Abführung für Kohlenwasserstoffe vorhanden ist,
- und wobei als nicht-Wasserstoffionen-leitende rohrförmige elektrochemische Zellen Sauerstoffionen-leitende rohrförmige elektrochemische Zellen vorhanden sind, wobei bei den Sauerstoffionen-leitenden rohrförmigen elektrochemischen Zellen auf der Kathodenseite eine Zuführung für Kohlenstoffdioxid und eine Abführung für Kohlenstoffmonoxid vorhanden ist,
- und wobei die mindestens eine Wasserstoffionen-leitende rohrförmige elektrochemische Zelle und die mindestens eine nicht-Wasserstoffionenleitende rohrförmige elektrochemische Zelle innerhalb eines Hüllrohres oder innerhalb unterschiedlicher Hüllrohre funktionell in Reihe und/oder parallel geschaltet sind.

2. Vorrichtung nach Anspruch 1, bei der 2 bis 1000 Wasserstoffionen-leitende rohrförmige elektrochemische Zellen als Bündel in einem Hüllrohr oder innerhalb mehrerer Hüllrohre zusammengefasst vorliegen, und/oder wobei mehrere Wasserstoffionen-leitende rohrförmige elektrochemische Zellen als Rohre oder Bündel über- und/oder hintereinander in einem Hüllrohrinnenraum angeordnet sind.

3. Vorrichtung nach Anspruch 1, bei der als nicht-Wasserstoffionen-leitende rohrförmige elektrochemische Zellen Sauerstoffionen-leitenden rohrförmige elektrochemische Zellen vorhanden sind, und ferner Wärmeübertrager oder Wärmetauscher zur Rekuperation von Abwärme exothermer Prozesse in den Hüllrohren, wie der Abwärme der Synthesereaktion, der Abwärme des Produktstromes, der Abwärme der Zwischenprodukte aus der Elektrolyse und/oder der Shift-Reaktion und/oder der Verdichterabwärme, und/oder Trennvorrichtungen, wie Vorrichtungen zur Trennung von Haupt- und Nebenprodukten, wie Wasser und nicht umgesetzte Edukte, beispielsweise durch Destillation, Kondensation oder Membranverfahren, oder zur Abtrennung von Wasserdampf und/oder Vorrichtungen zur Synthese und/oder zur Verarbeitung von Wasser, Wasserstoff, Kohlenstoffmonoxid und/oder Kohlenstoffdioxid vorhanden sind.

4. Vorrichtung nach Anspruch 2 oder 3, bei der eine oder mehrere Sauerstoffionen-leitenden rohrförmige elektrochemische Zellen in einem Hüllrohr mit einer oder mehreren Wasserstoffionen-leitenden, rohrförmigen elektrochemischen Zellen oder in mehreren Hüllrohren vorhanden sind.

5. Vorrichtung nach Anspruch 1, bei der das Hüllrohr aus metallischem Material besteht, vorteilhafterweise zur Aufnahme von Drücken im Hüllrohrinnenraum von 0,1 bis 40 MPa.

6. Vorrichtung nach Anspruch 1, bei der die mindestens eine Wasserstoffionenleitende rohrförmige elektrochemische Zelle Abmessungen von 1 mm bis 1000 mm Innendurchmesser und 20 mm bis 2000 mm Länge aufweist.

7. Vorrichtung nach Anspruch 1, bei der die elektrochemischen Zellen als Anodenmaterial einen Keramik-Metall-Verbunde (Cermet) aus Nickel-yttriumstabilisiertem Zirkonoxid, Nickellegierungen, Platinmetalle, oder Verbunde aus Oxidkeramiken und diesen Metallen aufweisen, und als Kathodenmaterial Lanthanstrontium-manganit, Lanthanstrontium-cobaltitferrit, Nickellegierungen oder Platinmetalle aufweisen, wobei mindestens die Innen- und Außenseiten des rohrförmigen Festelektrolyten mindestens teilweise, vorteilhafterweise vollständig, beschichtet sind.

8. Vorrichtung nach Anspruch 1, bei der bei den elektrochemischen Zellen Katalysatormaterial vorhanden ist, wobei jeweils als Katalysatormaterialien Platinmetalle, Nickel- und Eisenverbindungen für die Wasserstoffsynthese, und/oder für die Sauerstoffsynthese, und/oder nickel- oder rutheniumhaltige Materialien für die Methansynthese oder Kupfer-Zinkoxid-Aluminiumoxid-Materialien für die Methanolsynthese, oder kobalthaltige Katalysatoren generell für die Kohlenwasserstoffsynthese vorhanden sind.

9. Vorrichtung nach Anspruch 1, bei der als Heizelement ein oder mehrere elektrische Widerstandsheizelemente vorhanden sind.

10. Vorrichtung nach Anspruch 1, bei der einige oder alle Wasserstoffionen-leitenden rohrförmigen elektrochemischen Zellen und einige oder alle nicht-Wasserstoffionenleitenden rohrförmigen elektrochemischen Zellen funktionell parallel geschaltet sind und getrennte Zuführungen und Abführungen für die Ausgangs- und Endprodukte jeder Zelle aufweisen.

11. Vorrichtung nach Anspruch 1, bei der einige oder alle Wasserstoffionen-leitenden rohrförmigen elektrochemischen Zellen und einige oder alle nicht-Wasserstoffionenleitenden rohrförmigen elektrochemischen Zellen funktionell in Reihe geschaltet sind und die Zuführungen gleichzeitig die Abführungen oder die Abführungen gleichzeitig die Zuführungen anderer Zellen sind.

12. Verwendung der Vorrichtung nach Anspruch 1, bei der mindestens Wasserstoff synthetisiert wird, welches innerhalb des Hüllrohres zur Synthese von Kohlenwasserstoffen, wie Methan, Alkane, Alkene, Otto- oder Dieselkraftstoffe, oder eingesetzt wird.

## Claims

1. Apparatus for the synthesis of hydrogen-containing compounds,
- consisting of at least one shell tube which has, in the interior of the shell tube, at least one tubular electrochemical cell which conducts hydrogen ions and at least one tubular electrochemical cell which does not conduct hydrogen ions, which electrochemical cells are each arranged at a distance from the interior wall of the shell tube, where the tubular electrochemical cell which conducts hydrogen ions consists of a tubular solid electrolyte and a coating which consists of cathode or anode material is arranged on at least the interior wall and outer wall of the tubular solid electrolyte,
- or consisting of at least two shell tubes in which at least one tubular electrochemical cell which conducts hydrogen ions is present in one shell tube interior space and at least one tubular electrochemical cell which does not conduct hydrogen ions is present in the other shell tube interior space, where the two cells are each arranged at a distance from the interior wall of the shell tube and the tubular electrochemical cell which conducts hydrogen ions consists of a tubular solid electrolyte and a coating which consists of cathode or anode material is arranged on at least the interior wall and outer wall of the tubular solid electrolyte,
- where, at least in the operating state, a pressure greater than ambient pressure is present in the shell tube interior space of each shell tube
- and a heating element is present in the shell interior space of at least one shell tube
- and, at least in the tubular electrochemical cells which conduct hydrogen ions, the surfaces of the tubular solid electrolyte have a catalyst material at least on the cathode side and are completely or partly coated therewith and/or the anode and cathode material is, at least in part, a catalyst material and the catalyst material is positioned on the cathode material and/or anode material
- and, at least in the case of the tubular electrochemical cells which conduct hydrogen ions, a feed conduit for water or water vapour is present on the anode side of the cell and a feed conduit for carbon monoxide is present on the cathode side of the cell
- and, at least in the case of the tubular electrochemical cells which conduct hydrogen ions, a discharge conduit for oxygen is present on the anode side and a discharge conduit for hydrocarbons is present on the cathode side
- and tubular electrochemical cells which conduct oxygen ions are present as tubular electrochemical cells which do not conduct hydrogen ions, where in the case of the tubular electrochemical cells which conduct oxygen ions a feed conduit for carbon dioxide and a discharge conduit for carbon monoxide are present on the cathode side,
- and the at least one tubular electrochemical cell which conducts hydrogen ions and the at least one tubular electrochemical cell which does not conduct hydrogen ions are connected functionally in series and/or in parallel within a shell tube or within different shell tubes.

2. Apparatus according to Claim 1, wherein from 2 to 1000 tubular electrochemical cells which conduct hydrogen ions are present collected together as a bundle in a shell tube or within a plurality of shell tubes and/or a plurality of tubular electrochemical cells which conduct hydrogen ions are arranged as tubes or a bundle above one another and/or in succession in a shell tube interior space.

3. Apparatus according to Claim 1, wherein tubular electrochemical cells which conduct oxygen ions are present as tubular electrochemical cells which do not conduct hydrogen ions and heat transferers or heat exchangers for recuperating waste heat from exothermic processes, for example the waste heat of the synthesis reaction, the waste heat of the product stream, the waste heat of the intermediates from the electrolysis and/or the shift reaction and/or the compressor waste heat and/or separation devices, for example devices for separating main products and by-products such as water and unreacted starting materials, for example by distillation, condensation or membrane processes, or for separating off water vapour and/or apparatuses for synthesis and/or for processing of water, hydrogen, carbon monoxide and/or carbon dioxide, are additionally present in the shell tubes.

4. Apparatus according to Claim 2 or 3, wherein one or more tubular electrochemical cells which conduct oxygen ions are present in a shell tube with one or more tubular electrochemical cells which conduct hydrogen ions or in a plurality of shell tubes.

5. Apparatus according to Claim 1, wherein the shell tube consists of metallic material, advantageously for withstanding pressures in the shell tube interior space of from 0.1 to 40 MPa.

6. Apparatus according to Claim 1, wherein the at least one tubular electrochemical cell which conducts hydrogen ions has dimensions of from 1 mm to 1000 mm internal diameter and from 20 mm to 2000 mm length.

7. Apparatus according to Claim 1, wherein the electrochemical cells comprise a ceramic-metal composite (cermet) composed of nickel-yttrium-stabilized zirconium oxide, nickel alloys, platinum metals or composites of oxide ceramics and these metals as anode material and comprise lanthanum strontium manganite, lanthanum strontium cobaltite ferrite, nickel alloys or platinum metals as cathode material, with at least the insides and outsides of the tubular solid electrolyte being coated at least partly, advantageously completely.

8. Apparatus according to Claim 1, wherein catalyst material is present in the electrochemical cells, where platinum metals, nickel compounds and iron compounds for the synthesis of hydrogen and/or for the synthesis of oxygen, and/or nickel- or ruthenium-containing materials for the synthesis of methane or copper-zinc oxide-aluminium oxide materials for the synthesis of methanols, or cobalt-containing catalysts for the synthesis of hydrocarbons in general are in each case present as catalyst materials.

9. Apparatus according to Claim 1, wherein one or more electric resistance heating elements are present as heating element.

10. Apparatus according to Claim 1, wherein some or all tubular electrochemical cells which conduct hydrogen ions and some or all tubular electrochemical cells which do not conduct hydrogen ions are functionally connected in parallel and have separate feed conduits and discharge conduits for the starting materials and end products of each cell.

11. Apparatus according to Claim 1, wherein some or all tubular electrochemical cells which conduct hydrogen ions and some or all tubular electrochemical cells which do not conduct hydrogen ions are functionally connected in series and the feed conduits are at the same time the discharge conduits or the discharge conduits are at the same time the feed conduits of other cells.

12. Use of the apparatus according to Claim 1, wherein at least hydrogen which is used within the shell tube for the synthesis of hydrocarbons such as methane, alkanes, alkenes, spark-ignition fuels or diesel fuels is synthesized.

## Revendications

1. Dispositif pour la synthèse de composés contenant de l'hydrogène,
- constitué par au moins un tube de gainage, qui comprend dans l'espace intérieur du tube de gainage au moins une cellule électrochimique tubulaire conduisant les ions hydrogène et au moins une cellule électrochimique tubulaire conduisant des ions non-hydrogène, qui sont chacune agencées espacées de la paroi intérieure du tube de gainage, la cellule électrochimique tubulaire conduisant les ions hydrogène étant constituée par un électrolyte solide tubulaire, et un revêtement étant agencé au moins sur la paroi intérieure et extérieure de l'électrolyte solide tubulaire, qui est constitué par un matériau de cathode ou d'anode,
- ou constitué par au moins deux tubes de gainage, dans l'espace intérieur de tube de gainage d'un desquels est présente au moins une cellule électrochimique tubulaire conduisant les ions hydrogène et dans l'autre espace intérieur de tube de gainage est présente au moins une cellule électrochimique conduisant des ions non-hydrogène, les deux cellules étant chacune agencées espacées de la paroi intérieure du tube de gainage, et la cellule électrochimique tubulaire conduisant les ions hydrogène étant constituée par un électrolyte solide tubulaire, et un revêtement étant agencé au moins sur la paroi intérieure et extérieure de l'électrolyte solide tubulaire, qui est constitué par un matériau de cathode ou d'anode,
- une pression supérieure à la pression ambiante étant présente dans l'espace intérieur de tube de gainage de chaque tube de gainage au moins au stade de travail,
- et un élément chauffant étant présent dans l'espace intérieur de tube de gainage d'au moins un tube de gainage,
- et, au moins dans les cellules électrochimiques tubulaires conduisant les ions hydrogène, les surfaces de l'électrolyte solide tubulaire comprenant un matériau catalytique au moins sur le côté de la cathode et étant revêtues en totalité ou en partie avec celui-ci, et/ou le matériau d'anode et de cathode étant au moins en partie un matériau catalytique, et le matériau catalytique étant positionné sur le matériau de cathode et/ou d'anode,
- et, au moins dans les cellules électrochimiques tubulaires conduisant les ions hydrogène, une alimentation pour de l'eau ou de la vapeur d'eau étant présente sur le côté de l'anode de la cellule, et une alimentation pour du monoxyde de carbone étant présente sur le côté de la cathode de la cellule,
- et, au moins dans les cellules électrochimiques tubulaires conduisant les ions hydrogène, une sortie pour de l'oxygène étant présente sur le côté de l'anode, et une sortie pour des hydrocarbures sur le côté de la cathode,
- et des cellules électrochimiques tubulaires conduisant les ions oxygène étant présentes en tant que cellules électrochimiques tubulaires conduisant des ions non-hydrogène ; dans les cellules électrochimiques tubulaires conduisant les ions oxygène, une alimentation pour du dioxyde de carbone et une sortie pour du monoxyde de carbone étant présentes sur le côté de la cathode,
- et ladite au moins une cellule électrochimique tubulaire conduisant les ions hydrogène et ladite au moins une cellule électrochimique tubulaire conduisant des ions non-hydrogène étant raccordées fonctionnellement en série et/ou en parallèle dans un tube de gainage ou dans différents tubes de gainage.

2. Dispositif selon la revendication 1, dans lequel 2 à 1 000 cellules électrochimiques tubulaires conduisant les ions hydrogène sont rassemblées sous la forme d'un faisceau dans un tube de gainage ou dans plusieurs tubes de gainage, et/ou dans lequel plusieurs cellules électrochimiques tubulaires conduisant les ions hydrogène sont agencées sous la forme de tubes ou de faisceaux les unes sur les autres et/ou les unes après les autres dans un espace intérieur de tube de gainage.

3. Dispositif selon la revendication 1, dans lequel des cellules électrochimiques tubulaires conduisant les ions oxygène sont présentes en tant que cellules électrochimiques tubulaires conduisant des ions non-hydrogène, et des dispositifs de transfert de chaleur ou des échangeurs de chaleur sont en outre présents pour la récupération de la chaleur résiduaire de procédés exothermiques dans les tubes de gainage, telle que la chaleur résiduaire de la réaction de synthèse, la chaleur résiduaire du courant de produits, la chaleur résiduaire des produits intermédiaires de l'électrolyse et/ou de la réaction de conversion et/ou la chaleur résiduaire du compresseur, et/ou des dispositifs de séparation, tels que des dispositifs pour la séparation de produits principaux et secondaires, tels que l'eau et les réactifs non réagis, par exemple par distillation, condensation ou des procédés sur membrane, ou pour la séparation de vapeur d'eau, et/ou des dispositifs pour la synthèse et/ou pour le traitement d'eau, d'hydrogène, de monoxyde de carbone et/ou de dioxyde de carbone.

4. Dispositif selon la revendication 2 ou 3, dans lequel une ou plusieurs cellules électrochimiques tubulaires conduisant les ions oxygène sont présentes dans un tube de gainage avec une plusieurs cellules électrochimiques tubulaires conduisant les ions hydrogène ou dans plusieurs tubes de gainage.

5. Dispositif selon la revendication 1, dans lequel le tube de gainage est constitué par un matériau métallique, avantageusement pour l'absorption de pressions dans l'espace intérieur du tube de gainage de 0,1 à 40 MPa.

6. Dispositif selon la revendication 1, dans lequel ladite au moins une cellule électrochimique tubulaire conduisant les ions hydrogène présente des dimensions de 1 mm à 1 000 mm de diamètre intérieur et de 20 mm à 2 000 mm de longueur.

7. Dispositif selon la revendication 1, dans lequel les cellules électrochimiques comprennent en tant que matériau d'anode un composite céramique-métal (Cermet) à base d'oxyde de zirconium stabilisé par du nickel-yttrium, des alliages de nickel, des métaux du groupe du platine ou des composites de céramiques oxydées et de ces métaux, et en tant que matériau de cathode de la manganite de lanthane-strontium, de la cobaltite-ferrite de lanthane-strontium, des alliages de nickel ou des métaux du groupe du platine, au moins les côtés intérieurs et extérieurs de l'électrolyte solide tubulaire étant revêtus au moins en partie, avantageusement en totalité.

8. Dispositif selon la revendication 1, dans lequel un matériau catalytique est présent dans les cellules électrochimiques, des métaux du groupe du platine, des composés de nickel et de fer pour la synthèse d'hydrogène et/ou pour la synthèse d'oxygène, et/ou des matériaux contenant du nickel ou du ruthénium pour la synthèse de méthane ou des matériaux à base de cuivre-oxyde de zinc-oxyde d'aluminium pour la synthèse de méthanol, ou des catalyseurs contenant du cobalt en général pour la synthèse d'hydrocarbures étant respectivement présents en tant matériaux catalytiques.

9. Dispositif selon la revendication 1, dans lequel un ou plusieurs éléments chauffants résistifs électriques sont présents en tant qu'élément chauffant.

10. Dispositif selon la revendication 1, dans lequel certaines ou toutes les cellules électrochimiques tubulaires conduisant les ions hydrogène et certaines ou toutes les cellules électrochimiques tubulaires conduisant des ions non-hydrogène sont raccordées fonctionnellement en parallèle et comprennent des alimentations et des sorties séparées pour les produits de départ et finaux de chaque cellule.

11. Dispositif selon la revendication 1, dans lequel certaines ou toutes les cellules électrochimiques tubulaires conduisant les ions hydrogène et certaines ou toutes les cellules électrochimiques tubulaires conduisant des ions non-hydrogène sont raccordées fonctionnellement en série, et les alimentations sont simultanément les sorties ou les sorties sont simultanément les alimentations d'autres cellules.

12. Utilisation du dispositif selon la revendication 1, dans laquelle au moins de l'hydrogène est synthétisé, qui est utilisé dans le tube de gainage pour la synthèse d'hydrocarbures, tels que du méthane, des alcanes, des alcènes, des carburants automobiles ou diesel.
